# EUROPÄISCHE PATENTSCHRIFT

(11) **EP 3 883 534 B1**
(45) Veröffentlichungstag und Bekanntmachung des Hinweises auf die Patenterteilung: **06.05.2026**
(21) Anmeldenummer: 19782997.1
(22) Anmeldetag: 02.10.2019
(51) Int. Cl.: A61K 8/34, A61K 8/73, A61K 8/97, A61Q 19/00, A61K 8/92, A61K 8/03, A61K 8/06

(54) **ÖLHALTIGE GESICHTSPFLEGEZUBEREITUNG**
OILY FACIAL CARE PREPARATION
PRÉPARATION DE SOINS DU VISAGE CONTENANT DE L'HUILE

(30) Priorität: 21.11.2018 DE 102018219931
(43) Veröffentlichungstag der Anmeldung: 29.09.2021
(73) Patentinhaber: Beiersdorf AG, 22529 Hamburg (DE)
(72) Erfinder: DRUCKS, Anja, 22399 Hamburg (DE); THEEL, Katharina, 22113 Oststeinbek (DE); MÖLLGAARD, Svenja Lena, 22337 Hamburg (DE); HEINZ, Rene, 25451 Quickborn (DE); PETTERSEN, Cathinka, 20250 Hamburg (DE)
(74) Vertreter: Beiersdorf AG
(86) Internationale Anmeldenummer: PCT/EP2019/076679
(87) Internationale Veröffentlichungsnummer: WO 2020/104092

(56) Entgegenhaltungen:
- WO-A1-2004/014325
- WO-A2-2014/013420
- DE-A1- 102010 013 277
- DE-A1- 102010 039 535
- DE-C1- 4 100 490
- DE-T2- 69 303 588
- DATABASE GNPD [online] MINTEL; 23 June 2014 (2014-06-23), ANONYMOUS: "Anti-Ageing Anti-Fatigue Eye Contour Cream Gel", XP055647339, retrieved from https://www.gnpd.com/sinatra/recordpage/2508871/ Database accession no. 2508871
- DATABASE GNPD [online] MINTEL; 23 June 2014 (2014-06-23), ANONYMOUS: "Anti-Ageing Anti-Fatigue Eye Contour Cream Gel", XP055647339, retrieved from www.gnpd.com Database accession no. 2508871

## Beschreibung

Die vorliegende Erfindung beschreibt eine transparente ölhaltige Gesichtspflegezubereitung, die pflegende Komponenten enthält, die wasserlöslich sind.

Ölhaltige Gesichtspflegezubereitungen, insbesondere Gesichtsöle, sind an sich bekannt. Sie werden auf das Gesicht aufgetragen, oftmals anstelle einer pflegenden Creme. Da die Hauptkomponente ein Öl ist, in vielen Fällen wenigstens zum Teil ein natürliches Öl, wird der Gesichtshaut mit dem Öl eine Substanz zur Verfügung gestellt, die den Lipiden der Hautzellen verwandt ist. Gesichtsöle sind in der Regel transparente Zubereitungen. Die Transparenz dieser Produktform wird von den Verbrauchern sehr geschätzt, verbinden sie doch mit transparenten Zubereitungen die Vorstellung von Reinheit. Dies ist eine Eigenschaft, die für Gesichtsprodukte sehr wichtig ist, da es der Verbraucher bevorzugt, auf seine Gesichtshaut nur transparente, reine Produkte aufzutragen.

Die menschliche Haut übt als größtes Organ des Menschen zahlreiche lebenswichtige Funktionen aus. Mit durchschnittlich etwa 2 m² Oberfläche beim Erwachsenen kommt ihr eine herausragende Rolle als Schutz- und Sinnesorgan zu. Aufgabe dieses Organs ist es, mechanische, thermische, aktinische, chemische und biologische Reize zu vermitteln und abzuwehren. Außerdem kommt ihr eine bedeutende Rolle als Regulations- und Zielorgan im menschlichen Stoffwechsel zu.

Die Haut ist in Schichten aufgebaut, wobei sich grob die Schichten Oberhaut (Epidermis), Lederhaut und Unterhaut unterscheiden lassen. Die Epidermis ist die äußere Schicht und steht mit der Umwelt in Kontakt. Die unterste Zelllage der Epidermis wird auch Basalzellschicht genannt. Diese Zellschicht hat ihre Teilungsfähigkeit bewahrt und liefert ständig neue Zellen nach. Es sind in der Regel Keratinocyten, die mit ihrer Reifung an die Zelloberfläche gelangen. Während des Reifungsprozesses wird vermehrt Keratin gebildet, die Zellen verhornen und der Zellkern geht zugrunde. In der Hornschicht (Stratum corneum) sind abgestorbene Keratinocyten zu finden, die durch epidermale Lipide zusammengehalten werden. Es besteht die Vorstellung von einem mauerartigen Aufbau der Hornschicht, die abgestorbenen Keratinocyten stellen die Ziegel dar und die epidermalen Lipide den Mörtel. Die epidermalen Lipide werden überwiegend in den epidermalen Keratinocyten gebildet und in den Zellen in kleinen Vesikeln gespeichert, die auch als "lamellar bodies" bezeichnet werden. Ceramide, Cholesterol und freie Fettsäuren bilden im Wesentlichen die epidermalen Lipide, die in der Hornschicht zwischen den abgestorbenen Keratinocyten zu finden sind. Im äußeren, oberen Bereich der Hornschicht sind neben den genannten Lipiden auch Lipide der Talgdrüsen zu finden.

Talgdrüsen produzieren Talg, der sich aus folgenden Komponenten zusammensetzt: Triglyceride, freie Fettsäuren, Wachse, Squalen und Cholesterin. Neben den Talgdrüsen sind in der Haut Schweißdrüsen zu finden. Diese Drüsen sondern den Schweiß ab, der neben Wasser auch Salze, Harnstoff, Harnsäure, Aminosäuren, Fettsäuren, Ammoniak, Zucker, Milchsäure und Ascorbinsäure (Vitamin C) in geringen Mengen enthält. Talg und die Sekrete der Schweißdrüsen bilden den Hydrolipidfilm der Haut aus, der auch Hydrolipid-Emulsion genannt wird. Dieser Hydrolipidfilm hat mehrere Funktionen, u.a. die Verhinderung der Austrocknung der Haut.

Unter kosmetischer Hautpflege ist in erster Linie zu verstehen, die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) zu stärken oder wiederherzustellen, sowie ihre Hornschicht bei aufgetretenen Schäden in ihrem natürlichen Regenerationsvermögen zu unterstützen.

Werden die Barriereeigenschaften der Haut gestört, kann es zu verstärkter Resorption toxischer oder allergener Stoffe oder zum Befall von Mikroorganismen und als Folge zu toxischen, allergischen oder entzündlichen Hautreaktionen kommen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Um die Barriereeigenschaften der Haut zu stärken oder die Barrierefunktion der Haut zu unterstützen, werden Haut-pflegende Zubereitungen auf die Haut aufgetragen. In der Kosmetik ist eine Vielzahl unterschiedlicher Pflegeprodukte bekannt, so auch Gesichtsöle.

Gesichtsöle können im Wesentlichen aus Ölen zusammengesetzt sein, wie beispielsweise das Gesichtsöl Mandel der Firma Weleda (Mintel Nummer: 5405477).

Auch das Produkt Clarins Huile Santal (Mintel Nummer 1719026) besteht zum überwiegenden Teil aus Ölen; zusätzlich ist Parfüm enthalten.

Im Produkt Gesichtsöl, Clarifying Day Oil, Dr. Hauschka sind neben Ölen eine Reihe von Pflanzenextrakten und Parfüm enthalten.

Als weitere Gesichtsöle oder ölhaltige Zubereitungen können das Hydrating Oil Serum der Firma Decléor (Mintel Nummer 3876973), das SK-II Oil der Firma Procter & Gamble (Mintel Nummer 3893625), das Extraordinary Oil der Firma L'Oréal (Mintel Nummer 4088883), das Youth Oil Serum der Firma Decléor (Mintel Nummer 4452381), das Amara Hydrating Oil Serum der Firma Decléor (mintel Nummer 4952243) und das Evening Primrose Oil Facial Renewing Dry Oil der Firma LaScad (Mintel Nummer 5315795) genannt werden.

Aufgabe der vorliegenden Erfindung war es nun, ölhaltige Gesichtspflegezubereitungen zur Verfügung zu stellen, die neben den Ölen weitere pflegende Komponenten enthalten, die das Wirk- und/oder Pflegespektrum der ölhaltigen Gesichtspflegezubereitung erweitern. Um dies zu erreichen, wurde die Vorstellung entwickelt, polare oder polarere Komponenten einzuarbeiten, die mit dem Hydrolipidfilm der Haut wechselwirken oder ihn stärken könnten. Wie bereits beschrieben enthält der Hydrolipidfilm der Haut neben Lipiden auch Wasser und wasserlösliche Stoffe. Es stellte sich also die Aufgabe, ölhaltige Gesichtspflegezubereitungen bereit zu stellen, die zusätzlich zu den öligen Komponenten polare, wasserlösliche Verbindungen enthalten. In der Regel führt die Einarbeitung von polaren, wasserlöslichen Verbindungen in ölige Zusammensetzungen zu Eintrübungen. Trotzdem war es Ziel der vorliegenden Erfindung transparente Zubereitungen zur Verfügung zu stellen.

Gelöst wird diese Aufgabe durch eine kosmetische Zwei-Phasen-Zubereitung, aufweisend zwei getrennte Phasen, die visuell in eine obere und eine untere Phase unterscheidbar sind, wobei die eine Phase eine hydrophile Phase ist, enthaltend
- wenigstens einen wasserlöslichen Pflegestoff,
- wenigstens ein Polyol, ausgewählt aus der Gruppe Glycerin und/oder Propylenglycol wobei das wenigstens eine Polyol, ausgewählt aus der Gruppe Glycerin und/oder Propylenglycol, mit einem Gehalt von 70 bis 95 Gew. %, bevorzugt von 75 bis 90 Gew. % enthalten ist, bezogen auf das Gesamtgewicht der hydrophilen Phase und
- ein oder mehrere Konservierungsmittel und/oder ein oder mehrere die Konservierung unterstützende Substanzen enthalten sind, bevorzugt Caprylylglycol und/oder Benzethonium Chlorid
- Wasser und
   wobei die andere Phase eine Lipidphase ist, enthaltend
- wenigstens ein natürliches Pflanzenöl und
- optional wenigstens ein Konservierungsmittel ausgewählt aus Öl-löslichen Konservierungsmitteln.

Die erfindungsgemäßen zwei Phasen weisen charakteristischerweise einen deutlichen Unterschied in ihrer Dichte auf.

Dem Fachmann sind die Schriften DE102010039535, DE4100490, DE69303588T, WO2014/013420, WO2004/014325, DE1022010013277 und das Marktprodukt "Anti-Ageing Anit-Fatigue Eye Contour Cream Gel" (GNPD-Datenbank Eintragsnummer 2508871) bekannt, doch konnten diese Veröffentlichungen nicht den Weg zur vorliegenden Erfindung weisen.

Die kosmetische Zwei-Phasen-Zubereitung wird in ein geeignetes Packmittel gegeben. Vor Anwendung wird das Packmittel enthaltend die erfindungsgemäße 2-Phasen-Zubereitung geschüttelt, so dass sich beide Phasen vermischen und visuell eine weitgehend homogene Phase entsteht. Von dieser homogenen Phase wird eine geeignete Menge auf die Gesichtshaut aufgetragen und verteilt. Nach Gebrauch wird das Packmittel, enthaltend die gemischte Zwei-Phasen-Zubereitung, aufgestellt. Nun trennen sich die Phasen wieder. Dies geschieht in einem Zeitintervall von etwa 3 bis 15 Minuten, wobei nach dieser Zeit die Phasen weitgehend getrennt vorliegen. Eine vollständige Trennung wird nach 2 bis 3 Stunden erreicht.

Die erfindungsgemäße Zwei-Phasenzubereitung ist vorteilhaft als ölhaltige Gesichtspflegezubereitung für die Haut des menschlichen Gesichts zu verwenden. Diese Zubereitung ist vorteilhaft eine "leave-on"-Zubereitung, d.h. die Zubereitung bleibt nach der Anwendung auf der Haut, um ihre Wirkung zu entfalten.

Die erfindungsgemäße Zwei-Phasen-Zubereitung kann auch auf die Haut des gesamten menschlichen Körpers aufgetragen werden, also auch als Körperöl verwendet werden.

Die hydrophile Phase der erfindungsgemäßen Zwei-Phasen-Zubereitung enthält Wasser mit einem Anteil von 5 bis 25 Gew.-%, bevorzugt 10 bis 20 Gew.-%, bezogen auf das Gesamtgewicht der hydrophilen Phase.

Die hydrophile Phase der erfindungsgemäßen Zwei-Phasen-Zubereitung enthält einen wasserlöslichen Pflegestoff. Aus der Vielzahl an geeigneten wasserlöslichen Pflegestoffen haben sich natürliche Wirkstoffe und/oder deren Derivate als vorteilhaft erwiesen, wie z. B. alpha-Liponsäure, D-Biotin, alpha-Glucosylrutin, Carnitin, Carnosin, natürliche und/oder synthetische Isoflavonoide, Kreatin, Kreatinin, Taurin und/oder β-Alanin.

Ebenso vorteilhafte Pflegestoffe sind Stoffe, die sich von in der extrazellulären Matrix von Zellen vorhandenen Stoffen ableiten. Besonders vorteilhaft hat sich Hyaluronsäure oder das Salz der Hyaluronsäure, insbesondere das Natriumsalz der Hyaluronsäure, erwiesen. Hyaluronsäure ist ein lineares, langkettiges Polysaccharid, das aus sich wiederholenden Disacchariden aufgebaut ist. Die Disaccharide werden als Glucuronyl-β-(1→3)-*N-*Acetylglucosamin-Einheiten bezeichnet, die β-(1→4)-glycosidisch miteinander verknüpft sind. Es können bis 100.000 Disaccharideinheiten miteinander verknüpft sein.

Natrium-Hyaluronat kann beispielsweise von der Firma Inno Biotechnology bezogen werden.

Der wenigstens eine Pflegestoff liegt in der hydrophilen Phase der erfindungsgemäßen Zwei-Phasen-Zubereitung mit einem Gehalt von 0,01 bis 5,0 Gew.-%, bevorzugt 0,1 bis 2,0 Gew.-% vor, bezogen auf das Gesamtgewicht der hydrophilen Phase.

Weiterhin ist es vorteilhaft Extrakte in die hydrophile Phase einzuarbeiten, dies können zum einen wässrige pflanzliche Extrakte sein, wie beispielsweise ein Anisextrakt, Pimpinella Anisum Fruit Extract. Zum anderen können es auch Perlenextrakte sein, die als Puder vorliegen, die wasserlöslich sind. Dies ist beispielsweise Hydrolyzed Pearl Powder mit der INCI-Bezeichnung Pearl Powder.

Die hydrophile Phase der erfindungsgemäßen Zwei-Phasen-Zubereitung enthält wenigstens ein Polyol, ausgewählt aus der Gruppe Glycerin und/oder Propylenglycol.

Glycerin und/oder Polyol haben in kosmetischen Zubereitungen oftmals mehr als nur eine Funktion. In der erfindungsgemäßen Zubereitung tragen ein oder beide Komponenten zur Hautbefeuchtung bei. Zudem wirken sie als Lösungsvermittler.

Das wenigstens eine Polyol, ausgewählt aus der Gruppe Glycerin und/oder Propylenglycol ist in der hydrophilen Phase mit einem Gehalt von 70 bis 95 Gew.-%, bevorzugt von 75 bis 90 Gew.-% enthalten, bezogen auf das Gesamtgewicht der hydrophilen Phase.

In der hydrophilen Phase der erfindungsgemäßen Zwei-Phasen-Zubereitung ist/sind vorteilhaft zusätzlich ein oder mehrere Konservierungsmittel enthalten.

Denaturierter Alkohol (alcohol denat.), also vergällter Alkohol, lässt sich in die hydrophile Phase einarbeiten. Unter Alkohol wird in diesem Zusammenhang nur Ethanol verstanden.

In der hydrophilen Phase der erfindungsgemäßen Zwei-Phasen-Zubereitung ist der denaturierte Alkohol mit einem Gehalt von 2,0 bis 4,0 Gew.-%, bevorzugt 2,5 bis 3,5 Gew.-% enthalten, bezogen auf das Gesamtgewicht der hydrophilen Phase.

Als vorteilhaft hat sich die Einarbeitung von Benzethonium Chlorid erwiesen. Benzethonium Chlorid entfaltet eine konservierende Wirkung und wird in kosmetischen Zubereitungen als Konservierungsmittel eingesetzt. Zudem hat Benzethonium Chlorid in Zwei-Phasen-Zubereitungen, die vor Anwendung auf der Haut gemischt werden, so dass sich eine visuell weitgehend homogene Zubereitung bildet, die sich in Ruhe wieder in eine Zwei-Phasen-Zubereitung trennt, einen verzögernden Effekt auf die erneute Ausbildung der Zwei-Phasen-Zubereitung nach dem Mischen. Dies wird in den Anmeldungen DE 102012219641 A1 und DE 102015204662 A1 beschrieben.

In der hydrophilen Phase der erfindungsgemäßen Zwei-Phasen-Zubereitung ist Benzethonium Chlorid mit einem Gehalt von 0,005 bis 0,1 Gew.-%, bevorzugt von 0,01 bis 0,05 Gew.-% enthalten, bezogen auf das Gesamtgewicht der hydrophilen Phase.

Als ebenso vorteilhaft hat sich die Einarbeitung bestimmter die Konservierung der erfindungsmäßen Zubereitung unterstützender Substanzen erwiesen. Diese Substanzen sind bevorzugt Alkandiole und als besonders bevorzugt hat sich Caprylylglycol erwiesen. Auch geeignet ist beispielsweise Decandiol. In der hydrophilen Phase der erfindungsgemäßen Zwei-Phasen-Zubereitung ist Caprylylglycol mit einem Gehalt von 0,01 bis 1,0 Gew.-%, bevorzugt von 0,05 bis 0,75 Gew.-% enthalten, bezogen auf das Gesamtgewicht der hydrophilen Phase.

Insbesondere vorteilhaft ist der Einsatz von Caprylylglycol und Benzethonium Chlorid in der hydrophilen Phase. Weiter insbesondere vorteilhaft ist es, wenn das Gewichtsverhältnis von Caprylylglycol zu Benzethonium Chlorid von 4,0 : 1 bis 1 : 1 beträgt.

In der hydrophilen Phase der erfindungsgemäßen Zwei-Phasen-Zubereitung ist/sind vorteilhaft zusätzlich ein oder mehrere wasserlösliche(r) Farbstoff(e) enthalten. Die Farbstoffe können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Insbesondere vorteilhaft sind die folgenden Farbstoffe CI 15985 und/oder CI 16035.

Der oder die Farbstoffe sind in der hydrophilen Phase der erfindungsgemäßen Zwei-Phasen-Zubereitung mit einem Gehalt von 0,00001 bis 0,001 Gew.-%, bevorzugt 0,00005 bis 0,0005 Gew.-% enthalten, bezogen auf das Gesamtgewicht der hydrophilen Phase.

Alle Inhaltsstoffe der hydrophilen Phase addieren sich zu 100 Gew.-%.

Die Lipidphase der erfindungsgemäßen Zwei-Phasen-Zubereitung enthält wenigstens ein natürliches Pflanzenöl. Natürliche Pflanzenöle sind beispielsweise Triglycerinester gesättigter und/oder ungesättigter, verzweigter und/oder unverzweigter Alkancarbonsäuren einer Kettenlänge von 8 bis 24, insbesondere 12 bis 18 C-Atomen verstanden. Die Fettsäuretriglyceride können beispielsweise vorteilhaft gewählt werden aus der Gruppe Arganöl, Avocadoöl, Jojobaöl, Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl, Macadamianussöl und dergleichen mehr. Insbesondere vorteilhaft ist die Gruppe Avocadoöl, Jojobaöl und/oder Mandelöl, wobei alle Öle zusammen eingesetzt werden können, aber auch in Zweierkombinationen oder einzeln.

Das wenigstens eine natürliche Pflanzenöl ist in der Lipidphase der erfindungsgemäßen Zwei-Phasen-Zubereitung mit einem Gehalt von 0,1 bis 10,0 Gew.-%, bevorzugt 0,5 bis 5,0 Gew.-% enthalten, bezogen auf das Gesamtgewicht der Lipidphase.

In der Lipidphase der erfindungsgemäßen Zwei-Phasen-Zubereitung können vorteilhaft weitere Ölkomponenten enthalten sein, die gewählt werden können aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten einwertigen Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten einwertigen Alkoholen einer Kettenlänge von 3 bis 30 C-Atomen. Solche Esteröle können dann insbesondere vorteilhaft gewählt werden aus der Gruppe Octylpalmitat, Octylcocoat, Octylisostearat, Octyldodecey-Imyristat, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, Ethylhexylstearat, 2-Hexyldecylstearat, 2-Octyldodecylpalmitat, Stearylheptanoat, Oleyloleat, Oleylerucat, Erucyloleat, Erucylerucat und Tridecylstearat, sowie Gemische solcher Esteröle. Bevorzugt sind die Esteröle Ethylhexylstearat und Cetearylisononanoat, die jeweils einzeln oder in Kombination eingesetzt werden können.

In der Lipidphase der erfindungsgemäßen Zwei-Phasen-Zubereitung beträgt die Gesamtmenge an einem oder mehreren Esteröl(en) von 10 bis 50 Gew.-%, bevorzugt von 20 bis 40 Gew.-%, bezogen auf das Gesamtgewicht der Lipidphase.

Weiterhin ist es vorteilhaft, wenn die Lipidphase der erfindungsgemäßen Zwei-Phasen-Zubereitung gesättigte und/oder ungesättigte, verzweigte und/oder unverzweigte, einwertige Alkohole einer Kettenlänge von 3 bis 30 C-Atomen enthält. Insbesondere vorteilhaft sind verzweigte Alkohole, wie beispielsweise 2-Hexyldecanol, 2-Octyldodecanol, Isotridecanol und Isohexadecanol, wobei 2-Octyldodecanol besonders bevorzugt ist.

In der Lipidphase der erfindungsgemäßen Zwei-Phasen-Zubereitung beträgt die Gesamtmenge an einem oder mehreren der genannten Alkohole von 45 bis 70 Gew.-%, bevorzugt 50 bis 75 Gew.-%, bezogen auf das Gesamtgewicht der Lipidphase.

Weiterhin können in der Lipidphase Dialkylether und/oder Dialkylcarbonate enthalten sein sowie ausgewählte verzweigte und/oder unverzweigte Kohlenwasserstoffe, insbesondere ausgewählt aus Mineralöl, Paraffinöl, Squalan und Squalen, Polyolefine, hydrogenierte Polyisobutene und Isohexadecan.

Ebenso können Silikonöle, insbesondere flüssige Dimethicone, in der Lipidphase enthalten sein.

Die Lipidphase der erfindungsgemäßen Zwei-Phasen-Zubereitung ist vorteilhaft unkonserviert, d.h. es wird kein Konservierungsmittel zu der erfindungsgemäßen Zubereitung zugegeben.

Dennoch kann wenigstens ein Konservierungsmittel ausgewählt aus Öl-löslichen Konservierungsmitteln enthalten sein. Als ein geeignetes Öl-lösliches Konservierungsmittel kann Phenoxyethanol ausgewählt werden. Phenoxyethanol hat eine bakterizide Wirkung und wird vielfach in kosmetischen Produkten zur Konservierung eingesetzt.

Wenn ein Öl-lösliches Konservierungsmittel in der Lipidphase der erfindungsgemäßen Zwei-Phasen-Zubereitung enthalten ist, so liegt es mit einem Gehalt von 0,1 bis 2,0 Gew.-%, bevorzugt 0,5 bis 1,5 Gew.-% vor, bezogen auf das Gesamtgewicht der Lipidphase.

In der Lipidphase der erfindungsgemäßen Zwei-Phasen-Zubereitung kann/können vorteilhaft zusätzlich ein oder mehrere öllösliche/r Farbstoff(e) enthalten sein. Insbesondere vorteilhaft sind die folgenden Farbstoffe CI 61565 und/oder CI 60725.

Wenn in der Lipidphase der erfindungsgemäßen Zwei-Phasen-Zubereitung ein oder mehrere öl-ölsliche Farbstoffe enthalten sind, so liegen mit einem Gehalt von 0,00001 bis 0,001 Gew.-%, bevorzugt 0,00005 bis 0,0005 Gew.-% vor, bezogen auf das Gesamtgewicht der Lipidphase.

Alle Inhaltsstoffe der Lipidphase addieren sich zu 100 Gew.-%.

In der hydrophilen Phase und in der Lipidphase der erfindungsgemäßen Zwei-Phasen-Zubereitung können weitere kosmetische Wirk- und Hilfsstoffe enthalten, sofern sie keine nachteiligen Effekte, insbesondere keine Eintrübungen, auf die Zwei-Phasen-Zubereitung ausüben.

Um die Zwei-Phasen-Zubereitung herzustellen, werden die Lipidphase und die hydrophile Phase in einem geeigneten Packmittel zusammengegeben. Dabei kann die hydrophile Phase vorgelegt werden und die Lipidphase wird auf diese Phase gegeben wird. Es ist jedoch auch möglich die Lipidphase vorzulegen und anschließend die hydrophile Phase zuzugeben.

Zur Herstellung der erfindungsgemäßen Zwei-Phasen-Zubereitung liegen die hydrophile Phase und die Lipidphase in einem bestimmten Gewichtsverhältnis vor, nämlich hydrophile Phase zu Lipidphase 1 : 3 bis 3 : 1. Ist in der erfindungsgemäßen Zubereitung der Anteil der hydrophilen Phase höher als derjenige der Lipidphase, steht eine mehr Haut-befeuchtende Wirkung im Vordergrund. Ist in der erfindungsgemäßen Zubereitung der Anteil der Lipidphase höher, so steht eine mehr pflegende, regenerierende Wirkung im Vordergrund. Liegen die hydrophile Phase und die Lipidphase in einem Gewichtsverhältnis von 1:1 vor, so wird eine ausgewogene Wirkung von Hautbefeuchtung und Pflege erzielt.

Es wurden verschiedene Konservierungsmittel und die Konservierung unterstützende Substanzen untersucht in Hinblick darauf, ob sie einen Einfluss auf die Transparenz der Zwei-Phasen-Zubereitung, der hydrophilen Phase oder der Lipidphase haben und ob sie in ausreichendem Maße eine Konservierung bewirken. Dabei wurde zunächst die Transparenz evaluiert. Bei Proben (nur hydrophile), die ausreichend transparent waren, wurde anschließend eine Prüfung der mikrobiologischen Stabilität durchgeführt.

**Tabelle 1a:**

| **Basis-Zusammensetzung der hydrophilen Phase:** | |
|---|---|
| | |
| **INCI-Bezeichnung** | **[Gew.%]** |
| Creatine | 0,20 |
| CI 15985 | 0,60 |
| CI 16035 | 0,10 |
| 1-Methylhydantoin-2-Imide | 0,20 |
| Sodium Hyaluronate | 0,20 |
| Sodium Chloride | 0,20 |
| Glycerine* | variabel |
| Konservierungsmittel** | variabel |
| Aqua* | variabel |
| Gesamtgewicht | 100,00 |
| | |
| * variable Mengen um die verschiedenen Gehalte an Konservierungsmitteln auszugleichen; Glyceringehalt 67,0 bis 82,5 Gew.-% | |
| ** Konservierungsmittel oder die Konservierung unterstützende Verbindung gemäß Tabelle 1b | |

**Tabelle 1b:**

| | Benzethonium Chlorid | Polyamino Biguanide | Alcohol denat. | Caprylyl Glycol | Piroctone Olamine | Phenoxyethanol | Methylparaben | Ethylparaben | Tranparenz der Lösung | Mikrobiologische Stabilität |
|---|---|---|---|---|---|---|---|---|---|---|
| | | | | | | | | | | |
| 1 | 0,08 | | | | | | | | ja | nein |
| 2 | 0,06 | | 3,00 | | | | | | ja | ja |
| 3 | 0,06 | 0,80 | 3,00 | | | | | | nein | |
| 4 | 0,06 | 0,80 | | | | | | | nein | |
| 5 | 0,06 | | 6,00 | | | | | | nein | |
| 6 | 0,06 | | 8,00 | | | | | | nein | |
| 7 | 0,06 | | 10,00 | | | | | | nein | |
| 8 | 0,03 | | | 0,10 | | | | | ja | ja |
| 9 | 0,03 | | | | 0,02 | | | | nein | |
| 10 | 0,03 | | | | | | 0,1 | | nein | |
| 11 | 0,03 | | | | | | | 0,1 | nein | |

**Tabelle 2a:**

| **Basis-Zusammensetzung der Lipidphase:** | |
|---|---|
| | |

| **INCI-Bezeichnung** | **[Gew.%]** |
|---|---|
| Persea Gratissima Oil | 0,50 |
| Octyldodecanol* | variabel |
| Simmondsia Chinensis Oil | 3,50 |
| Prunus Amygdalus Dulcis Oil | 0,50 |
| Ethylhexyl Stearate | 5,00 |
| Perfume | 0,60 |
| Cetearyl Isononanoate | 30,00 |
| Konservierungsmittel** | variabel |
| Gesamtgewicht | 100,00 |
| | |
| * variable Mengen um die verschiedenen Gehalte an Konservierungsmittel auszugleichen | |
| ** Konservierungsmittel gemäß Tabelle 2b oder auch kein Konservierungsmittel | |

**Tabelle 2b:**

| **Evaluierung von Konservierungsmitteln in der Lipidphase in Bezug auf die Transparenz der Lösung und in Bezug auf die mikrobiologische Stabilität:** | | | |
|---|---|---|---|
| | ohne Konservierungsmittel | Phenoxyethanol | Transparenz der Lösung |
| | | | |
| 12 | | 0,8 | ja |
| 13 | | 1,0 | ja |
| 14 | ja | | ja |

**Tabelle 3:**

| **Evaluierung von Konservierungsmitteln in der Zwei-Phasen-Zubereitung in Bezug auf die Transparenz der Lösung und in Bezug auf die mikrobiologische Stabilität:** | | | | | | | |
|---|---|---|---|---|---|---|---|
| | Benzethonium Chlorid | Polyamino Biguanide | Alcohol denat. | Caprylyl Glycol | Phenoxyethanol | Transpa-renz der Lösung | Mikrobiolo-gische Stabilität |
| | | | | | | | |
| 1 + 14 | 0,04 | | | | | ja | nein |
| 2 + 14 | 0,04 | | 1,5 | | | nein | |
| 3 + 14 | 0,04 | 0,4 | 1,5 | | | nein | |
| 4 + 14 | 0,08 | 0,8 | | | | nein | |
| 1 + 12 | 0,03 | | | | 0,4 | nein | |
| 1 + 13 | 0,03 | | | | 0,5 | nein | |
| 8 + 14 | 0,015 | | | 0,05 | | ja | ja |

Tabelle 3 zeigt ausgewählte Zwei-Phasen-Zubereitungen an. Für die Herstellung dieser Zubereitungen wurde jeweils eine hydrophile Phase gemäß einer Nummer aus Tabelle 1a + b mit einer Lipidphase gemäß einer Nummer aus Tabelle 2a + b zusammengegeben. Das Mischungsverhältnis war in jedem Fall ein Gewichtsverhältnis von hydrophiler Phase zu Lipidphase von 1:1.

Aus den oben stehenden Tabellen wird deutlich, dass einige der üblicherweise in der kosmetischen Industrie eingesetzten Konservierungsmittel oder Konservierungsmittelkombinationen die Transparenz insbesondere der hydrophilen Phase nachteilig beeinflussen.

Für die hydrophile Phase hat sich der Einsatz von Benzethonium Chlorid und denaturiertem Alkohol als geeignet erwiesen, wobei ein Gehalt an denaturiertem Alkohol von etwa 3,0 Gew.-% am vorteilhaftesten ist. Auch der Einsatz von Caprylyl Glycol zusammen mit Benzethonium Chlorid führt zu transparenten hydrophilen Phasen.

Für die Lipidphase wurde der Einsatz von Phenoxyethanol evaluiert und Phenoxyethanol hat sich als vorteilhaftes Konservierungsmittel für die Lipidphase erwiesen.

Für die Zwei-Phasen-Zubereitung hat sich der Einsatz von Benzethonium Chlorid zusammen mit Caprylyl Glycol in der hydrophilen Phase und der Verzicht auf Konservierungsmittel in der Lipidphase als günstig erwiesen.

Somit konnte gezeigt werden, dass es vorteilhafte Kombinationen von Konservierungsmitteln und die Konservierung unterstützenden Substanzen gibt, die die Bereitstellung einer transparenten ölhaltigen Gesichtspflegezubereitung in Form einer Zwei-Phasen-Zubereitung gewährleisten.

Auch Gegenstand der vorliegenden Erfindung ist die Verwendung von Benzethonium Chlorid zusammen mit denaturiertem Alkohol und/oder Caprylyl Glycol zur Bereitstellung einer transparenten hydrophilen Phase, die Teil einer Zwei-Phasen-Zubereitung ist, die zwei getrennte Phasen aufweist, die visuell in eine obere und eine untere Phase unterscheidbar sind, wobei die hydrophile Phase wenigstens einen wasserlöslichen Pflegestoff, wenigstens ein Polyol, ausgewählt aus der Gruppe Glycerin und/oder Propylenglycol und Wasser enthält, wobei die Lipidphase wenigstens ein natürliches Pflanzenöl enthält.

Weiterhin Gegenstand der vorliegenden Erfindung ist die Verwendung von Benzethonium Chlorid zusammen mit Caprylyl Glycol zur Bereitstellung einer transparenten, hydrophilen Phase, die Teil einer Zwei-Phasen-Zubereitung ist, die zwei getrennte Phasen aufweist, die visuell in eine obere und eine untere Phase unterscheidbar sind,
wobei die hydrophile Phase wenigstens einen wasserlöslichen Pflegestoff, wenigstens ein Polyol, ausgewählt aus der Gruppe Glycerin und/oder Propylenglycol und Wasser enthält, wobei die Lipidphase wenigstens ein natürliches Pflanzenöl enthält und wobei das Gewichtsverhältnis von Caprylyl Glycol zu Benzethonium Chlorid von 4,0 : 1 bis 1 : 1 beträgt.

Ebenso Gegenstand der vorliegenden Erfindung ist die Verwendung von Benzethonium Chlorid zusammen mit Caprylyl Glycol in einer hydrophilen Phase und einer Lipidphase ohne Konservierungsmittel,
wobei die hydrophile Phase und die Lipidphase Teile einer Zwei-Phasen-Zubereitung sind, zur Bereitstellung einer transparenten Zwei-Phasen-Zubereitung,
wobei die Zwei-Phasen-Zubereitung zwei getrennte Phasen aufweist, die visuell in eine obere und eine untere Phase unterscheidbar sind,
wobei die hydrophile Phase wenigstens einen wasserlöslichen Pflegestoff, wenigstens ein Polyol, ausgewählt aus der Gruppe Glycerin und/oder Propylenglycol und Wasser enthält und
wobei die Lipidphase wenigstens ein natürliches Pflanzenöl enthält.

### Beispiele:

Die Beispielrezepturen sollen die Erfindung verdeutlichen, ohne sie einzuschränken. Die Gewichtsangaben beziehen sich auf Gewichtsprozentangaben und Aktivgehalte, sofern nicht anders angegeben.

| **Hydrophile Phasen** | **1** | **2** | **3** | **4** |
|---|---|---|---|---|
| | | | | |

| INCI-Bezeichnung | [Gew.%] | [Gew.%] | [Gew.%] | [Gew.%] |
|---|---|---|---|---|
| | | | | |
| Sodium Chloride | 0,20 | 0,20 | 0,20 | 0,20 |
| Glycerine | 83,20 | 75,97 | 78,97 | 78,97 |
| Creatine | 0,20 | | | |
| 1-Methylhydantoin-2-Imide | 0,20 | | | |
| Sodium Hyaluronate | 0,20 | 0,20 | | |
| Carnitine | | | | 0,10 |
| Hydrolyzed Pearl | | | | 0,10 |
| Pimpinella Anisum Fruit Extract* | | 8,00 | | |
| Calcium Panthothenate | | | 0,10 | |
| Benzethonium Chloride | 0,06 | 0,03 | 0,03 | 0,03 |
| Caprylylglycol | 0,10 | 0,10 | 0,10 | 0,10 |
| CI 15985 | 0,60 | | | |
| CI 16035 | 0,10 | | | |
| Aqua | ad 100 | ad 100 | ad 100 | ad 100 |

| | | | | |
|---|---|---|---|---|
| * Rohstoffgehalt | | | | |

| **Lipidphasen:** | **1** | **2** | **3** | **4** |
|---|---|---|---|---|
| INCI-Bezeichnung | [Gew.%] | | | |
| Persea Gratissima Oil | 0,50 | | | |
| Simmondsia Chinensis Oil | 3,50 | 3,50 | 2,50 | |
| Prunus Amygdalus Dulcis Oil | 0,50 | 0,50 | | |
| Helianthus Annuus Seed Oil | | 0,50 | 4,50 | |
| Argania Spinosa Kernel Oil | | | 0,50 | |
| Vitis Vinifera Grape Seed Oil | | | | 0,50 |
| Ethylhexyl Stearate | 5,00 | 5,00 | 7,50 | 5,00 |
| Cetearyl Isononanoate | 30,00 | 30,00 | 25,00 | 35,00 |
| Perfume | 0,60 | 0,80 | 0,65 | 0,75 |
| Octyldodecanol | ad 100 | ad 100 | ad 100 | ad 100 |

Zur Herstellung der erfindungsgemäßen Zwei-Phasen-Zubereitung wird jeweils eine der hydrophilen Phasen und eine der Lipidphasen zusammengegeben und zwar in einem Gewichtsverhältnis von hydrophile Phase zu Lipidphase von 1 : 3 bis 3 : 1.

## Patentansprüche

1. Kosmetische Zwei-Phasen-Zubereitung, aufweisend zwei getrennte Phasen, die visuell in eine obere und eine untere Phase unterscheidbar sind,
wobei die eine Phase eine hydrophile Phase ist, enthaltend
- wenigstens einen wasserlöslichen Pflegestoff,
- wenigstens ein Polyol, ausgewählt aus der Gruppe Glycerin und/oder Propylenglycol
wobei das wenigstens eine Polyol, ausgewählt aus der Gruppe Glycerin und/oder Propylenglycol, mit einem Gehalt von 70 bis 95 Gew. %, bevorzugt von 75 bis 90 Gew. % enthalten ist, bezogen auf das Gesamtgewicht der hydrophilen Phase und
- ein oder mehrere Konservierungsmittel und/oder ein oder mehrere die Konservierung unterstützende Substanzen enthalten sind, bevorzugt Caprylylglycol und/oder Benzethonium Chlorid,
- Wasser,
wobei die andere Phase eine Lipidphase ist, enthaltend
- wenigstens ein natürliches Pflanzenöl und
- optional wenigstens ein Konservierungsmittel ausgewählt aus Öl-löslichen Konservierungsmitteln.

2. Zubereitung nach Anspruch 1, **dadurch gekennzeichnet, dass** die hydrophile Phase der Zwei-Phasen-Zubereitung Wasser mit einem Anteil von 5 bis 25 Gew.-%, bevorzugt 10 bis 20 Gew.-% enthält, bezogen auf das Gesamtgewicht der hydrophilen Phase.

3. Zubereitung nach Anspruch 1 und/oder 2, **dadurch gekennzeichnet, dass** der wenigstens eine wasserlösliche Pflegestoff ausgewählt wird aus der Gruppe alpha-Liponsäure, D-Biotin, alpha-Glucosylrutin, Carnitin, Carnosin, natürliche und/oder synthetische Isoflavonoide, Kreatin, Kreatinin, Taurin und/oder β-Alanin.

4. Zubereitung nach Anspruch 1 und/oder 2, **dadurch gekennzeichnet, dass** der wenigstens eine wasserlösliche Pflegestoff ausgewählt wird aus Hyaluronsäure, insbesondere dem Natriumsalz der Hyaluronsäure.

5. Zubereitung nach Anspruch 1 und/oder 2, **dadurch gekennzeichnet, dass** eine Kombination von wasserlöslichen Pflegestoffen aus Kreatin, Kreatinin und dem Natriumsalz der Hyaluronsäure enthalten ist.

6. Zubereitung nach wenigstens einem der vorstehenden Ansprüche, **dadurch gekennzeichnet, dass** in der hydrophilen Phase der Zwei-Phasen-Zubereitung der wenigstens eine Pflegstoff mit einem Gehalt von 0,01 bis 5,0 Gew.-%, bevorzugt 0,1 bis 2,0 Gew.-% vorliegt, bezogen auf das Gesamtgewicht der hydrophilen Phase.

7. Zubereitung nach wenigstens einem der vorstehenden Ansprüche, **dadurch gekennzeichnet, dass** in der hydrophilen Phase der Zwei-Phasen-Zubereitung Benzethonium Chlorid mit einem Gehalt von 0,005 bis 0,1 Gew.-%, bevorzugt von 0,01 bis 0,05 Gew.-% enthalten ist, bezogen auf das Gesamtgewicht der hydrophilen Phase.

8. Zubereitung nach wenigstens einem der vorstehenden Ansprüche, **dadurch gekennzeichnet, dass** zusätzlich in der hydrophilen Phase der Zwei-Phasen-Zubereitung ein oder mehrere die Konservierung unterstützende Substanzen enthalten sind, bevorzugt Caprylyl Glycol.

9. Zubereitung nach wenigstens einem der vorstehenden Ansprüche, **dadurch gekennzeichnet, dass** in der hydrophilen Phase der Zwei-Phasen-Zubereitung Caprylyl Glycol mit einem Gehalt von 0,01 bis 1,0 Gew.-%, bevorzugt von 0,05 bis 0,75 Gew.-% enthalten ist, bezogen auf das Gesamtgewicht der hydrophilen Phase.

10. Zubereitung nach wenigstens einem der vorstehenden Ansprüche, **dadurch gekennzeichnet, dass** das Gewichtsverhältnis von Caprylylglycol zu Benzethonium Chlorid von 4,0 : 1 bis 1 : 1 beträgt.

11. Zubereitung nach wenigstens einem der vorstehenden Ansprüche, **dadurch gekennzeichnet, dass** in der Lipidphase der Zwei-Phasen-Zubereitung das wenigstens eine natürliche Pflanzenöl ausgewählt wird aus der Gruppe Arganöl, Avocadoöl, Jojobaöl, Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl und/oder Macadamianussöl, insbesondere aus der Avocadoöl, Jojobaöl und/oder Mandelöl.

12. Zubereitung nach wenigstens einem der vorstehenden Ansprüche, **dadurch gekennzeichnet, dass** in der Lipidphase der Zwei-Phasen-Zubereitung das wenigstens eine natürliche Pflanzenöl mit einem Gehalt von 0,1 bis 10,0 Gew.-%, bevorzugt 0,5 bis 5,0 Gew.-% enthalten ist, bezogen auf das Gesamtgewicht der Lipidphase.

13. Zubereitung nach wenigstens einem der vorstehenden Ansprüche, **dadurch gekennzeichnet, dass** in der Lipidphase der Zwei-Phasen-Zubereitung zusätzlich Esteröle enthalten sind, bevorzugt Esteröle ausgewählt aus der Gruppe Octylpalmitat, Octylcocoat, Octylisostearat, Octyldodeceylmyristat, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, Ethylhexylstearat, 2-Hexyldecylstearat, 2-Octyldodecylpalmitat, Stearylheptanoat, Oleyloleat, Oleylerucat, Erucyloleat, Erucylerucat und Tridecylstearat, sowie Gemische davon, insbesondere bevorzugt ausgewählt Ethylhexylstearat und/oder Cetearylisononanoat.

14. Zubereitung nach wenigstens einem der vorstehenden Ansprüche, **dadurch gekennzeichnet, dass** in der Lipidphase der Zwei-Phasen-Zubereitung die Gesamtmenge an einem oder mehreren Esteröl(en) von 10,0 bis 50,0 Gew.-% beträgt, bevorzugt von 20 bis 40 Gew.-%, bezogen auf das Gesamtgewicht der Lipidphase.

15. Zubereitung nach wenigstens einem der vorstehenden Ansprüche, **dadurch gekennzeichnet, dass** in der Lipidphase der Zwei-Phasen-Zubereitung zusätzlich wenigstens ein gesättigter und/oder ungesättigter, verzweigter und/oder unverzweigter, einwertiger Alkohol mit einer Kettenlänge von 3 bis 30 C-Atomen enthält, bevorzugt wenigstens ein verzweigter, einwertiger Alkohol mit einer Kettenlänge von 3 bis 30 C-Atomen, insbesondere bevorzugt Octyldodecanol.

16. Zubereitung nach wenigstens einem der vorstehenden Ansprüche, **dadurch gekennzeichnet, dass** in der Lipidphase der Zwei-Phasen-Zubereitung der Gehalt des wenigstens einen gesättigten und/oder ungesättigten, verzweigten und/oder unverzweigten, einwertigen Alkohols mit einer Kettenlänge von 3 bis 30 C-Atomen von 45,0 bis 65,0 Gew.-%, bevorzugt 50,0 bis 60,0 Gew.-% beträgt, bezogen auf das Gesamtgewicht der Lipidphase.

17. Zubereitung nach wenigstens einem der vorstehenden Ansprüche, **dadurch gekennzeichnet, dass** in der Zwei-Phasen-Zubereitung die hydrophile Phase und die Lipidphase in einem Gewichtsverhältnis von hydrophiler Phase zu Lipidphase von 1 : 3 bis 3 : 1 vorliegen.

18. Verwendung von Benzethonium Chlorid zusammen mit Caprylyl Glycol in einer hydrophilen Phase und von einer unkonservierten Lipidphase,
wobei die hydrophile Phase und die Lipidphase Teile einer Zwei-Phasen-Zubereitung sind,
zur Bereitstellung einer transparenten Zwei-Phasen-Zubereitung,
wobei die Zwei-Phasen-Zubereitung zwei getrennte Phasen aufweist, die visuell in eine obere und eine untere Phase unterscheidbar sind,
wobei die hydrophile Phase
- wenigstens einen wasserlöslichen Pflegestoff,
- wenigstens ein Polyol, ausgewählt aus der Gruppe Glycerin und/oder Propylenglycol und
- Wasser enthält und
wobei die Lipidphase
- wenigstens ein natürliches Pflanzenöl enthält.

## Claims

1. Cosmetic two-phase preparation, comprising two separate phases which can be visually distinguished into an upper and a lower phase,
wherein one phase is a hydrophilic phase, comprising
- at least one water-soluble care substance,
- at least one polyol selected from the group of glycerin and/or propylene glycol,
wherein the at least one polyol selected from the group of glycerin and/or propylene glycol is present at a content of 70% to 95% by weight, preferably of 75% to 90% by weight, based on the total weight of the hydrophilic phase and
- one or more preservatives and/or one or more preservation-supporting substances are present, preferably caprylyl glycol and/or benzethonium chloride,
- water,
wherein the other phase is a lipid phase, comprising
- at least one natural vegetable oil and
- optionally at least one preservative selected from oil-soluble preservatives.

2. Preparation according to Claim 1, **characterized in that** the hydrophilic phase of the two-phase preparation comprises water at a proportion of 5% to 25% by weight, preferably 10% to 20% by weight, based on the total weight of the hydrophilic phase.

3. Preparation according to Claim 1 and/or 2, **characterized in that** the at least one water-soluble care substance is selected from the group of alpha-lipoic acid, D-biotin, alpha-glucosylrutin, carnitine, carnosine, natural and/or synthetic isoflavonoids, creatine, creatinine, taurine and/or β-alanine.

4. Preparation according to Claim 1 and/or 2, **characterized in that** the at least one water-soluble care substance is selected from hyaluronic acid, in particular the sodium salt of hyaluronic acid.

5. Preparation according to Claim 1 and/or 2, **characterized in that** a combination of water-soluble care substances from creatine, creatinine and the sodium salt of hyaluronic acid is present.

6. Preparation according to at least one of the preceding claims, **characterized in that** the at least one care substance is present in the hydrophilic phase of the two-phase preparation at a content of 0.01% to 5.0% by weight, preferably 0.1% to 2.0% by weight, based on the total weight of the hydrophilic phase.

7. Preparation according to at least one of the preceding claims, **characterized in that** benzethonium chloride is present in the hydrophilic phase of the two-phase preparation at a content of 0.005% to 0.1% by weight, preferably of 0.01% to 0.05% by weight, based on the total weight of the hydrophilic phase.

8. Preparation according to at least one of the preceding claims, **characterized in that** one or more preservation-supporting substances are additionally present in the hydrophilic phase of the two-phase preparation, preferably caprylyl glycol.

9. Preparation according to at least one of the preceding claims, **characterized in that** caprylyl glycol is present in the hydrophilic phase of the two-phase preparation at a content of 0.01% to 1.0% by weight, preferably of 0.05% to 0.75% by weight, based on the total weight of the hydrophilic phase.

10. Preparation according to at least one of the preceding claims, **characterized in that** the weight ratio of caprylyl glycol to benzethonium chloride is from 4.0:1 to 1:1.

11. Preparation according to at least one of the preceding claims, **characterized in that** in the lipid phase of the two-phase preparation the at least one natural vegetable oil is selected from the group of argan oil, avocado oil, jojoba oil, olive oil, sunflower oil, soyabean oil, peanut oil, rapeseed oil, almond oil, palm oil, coconut oil, castor oil, wheat germ oil, grape seed oil, safflower oil, evening primrose oil and/or macadamia nut oil, in particular from avocado oil, jojoba oil and/or almond oil.

12. Preparation according to at least one of the preceding claims, **characterized in that** the at least one natural vegetable oil is present in the lipid phase of the two-phase preparation at a content of 0.1% to 10.0% by weight, preferably 0.5% to 5.0% by weight, based on the total weight of the lipid phase.

13. Preparation according to at least one of the preceding claims, **characterized in that** ester oils are additionally present in the lipid phase of the two-phase preparation, preferably ester oils selected from the group of octyl palmitate, octyl cocoate, octyl isostearate, octyldodecyl myristate, cetearyl isononanoate, isopropyl myristate, isopropyl palmitate, isopropyl stearate, isopropyl oleate, n-butyl stearate, n-hexyl laurate, n-decyl oleate, isooctyl stearate, isononyl stearate, isononyl isononanoate, 2-ethylhexyl palmitate, 2-ethylhexyl laurate, ethylhexyl stearate, 2-hexyldecyl stearate, 2-octyldodecyl palmitate, stearyl heptanoate, oleyl oleate, oleyl erucate, erucyl oleate, erucyl erucate and tridecyl stearate, and mixtures thereof, especially preferably selected from ethylhexyl stearate and/or cetearyl isononanoate.

14. Preparation according to at least one of the preceding claims, **characterized in that** the total amount of one or more ester oil(s) in the lipid phase of the two-phase preparation is from 10.0% to 50.0% by weight, preferably from 20% to 40% by weight, based on the total weight of the lipid phase.

15. Preparation according to at least one of the preceding claims, **characterized in that** in the lipid phase of the two-phase preparation additionally comprises at least one saturated and/or unsaturated, branched and/or unbranched, monohydric alcohol having a chain length of 3 to 30 carbon atoms, preferably at least one branched, monohydric alcohol having a chain length of 3 to 30 carbon atoms, especially preferably octyldodecanol.

16. Preparation according to at least one of the preceding claims, **characterized in that** the content of the at least one saturated and/or unsaturated, branched and/or unbranched, monohydric alcohol having a chain length of 3 to 30 carbon atoms in the lipid phase of the two-phase preparation is from 45.0% to 65.0% by weight, preferably 50.0% to 60.0% by weight, based on the total weight of the lipid phase.

17. Preparation according to at least one of the preceding claims, **characterized in that** the hydrophilic phase and the lipid phase are present in the two-phase preparation in a weight ratio of hydrophilic phase to lipid phase of 1:3 to 3:1.

18. Use of benzethonium chloride together with caprylyl glycol in a hydrophilic phase and of an unpreserved lipid phase,
wherein the hydrophilic phase and the lipid phase are parts of a two-phase preparation, to provide a transparent two-phase preparation,
wherein the two-phase preparation comprises two separate phases which can be visually distinguished into an upper and a lower phase,
wherein the hydrophilic phase comprises
- at least one water-soluble care substance,
- at least one polyol selected from the group of glycerin and/or propylene glycol and
- water and
wherein the lipid phase comprises
- at least one natural vegetable oil.

## Revendications

1. Préparation cosmétique à deux phases présentant deux phases séparées qui peuvent être distinguées visuellement en une phase supérieure et une phase inférieure,
ladite une phase étant une phase hydrophile contenant
- au moins une substance de soin soluble dans l'eau,
- au moins un polyol choisi dans le groupe constitué par le glycérol et/ou le propylèneglycol
ledit au moins un polyol, choisi dans le groupe constitué par le glycérol et/ou le propylèneglycol, étant contenu en une teneur de 70 à 95% en poids, de préférence de 75 à 90% en poids, par rapport au poids total de la phase hydrophile et
- un ou plusieurs conservateurs et/ou une ou plusieurs substances favorisant la conservation étant contenu(e)(s), de préférence du caprylylglycol et/ou du chlorure de benzéthonium,
- de l'eau,
l'autre phase étant une phase lipidique contenant
- au moins une huile végétale naturelle et
- éventuellement au moins un conservateur choisi parmi les conservateurs solubles dans l'huile.

2. Préparation selon la revendication 1, **caractérisée en ce que** la phase hydrophile de la préparation à deux phases contient de l'eau en une proportion de 5 à 25% en poids, de préférence de 10 à 20% en poids, par rapport au poids total de la phase hydrophile.

3. Préparation selon la revendication 1 et/ou 2, **caractérisée en ce que** ladite au moins une substance de soin soluble dans l'eau est choisie dans le groupe acide alpha-lipoïque, D-biotine, alpha-glucosylrutine, carnitine, carnosine, isoflavonoïdes naturels et/ou synthétiques, créatine, créatinine, taurine et/ou β-alanine.

4. Préparation selon la revendication 1 et/ou 2, **caractérisée en ce que** ladite au moins une substance de soin soluble dans l'eau est choisie parmi l'acide hyaluronique, en particulier le sel sodique de l'acide hyaluronique.

5. Préparation selon la revendication 1 et/ou 2, **caractérisée en ce qu'**elle contient une combinaison de substances de soin solubles dans l'eau constituée par de la créatine, de la créatinine et du sel sodique de l'acide hyaluronique.

6. Préparation selon au moins l'une des revendications précédentes, **caractérisée en ce que** ladite au moins une substance de soin est présente dans la phase hydrophile de la préparation à deux phases en une teneur de 0,01 à 5,0% en poids, de préférence de 0,1 à 2,0% en poids, par rapport au poids total de la phase hydrophile.

7. Préparation selon au moins l'une des revendications précédentes, **caractérisée en ce que** la phase hydrophile de la préparation à deux phases contient du chlorure de benzéthonium en une teneur de 0,005 à 0,1% en poids, de préférence de 0,01 à 0,05% en poids, par rapport au poids total de la phase hydrophile.

8. Préparation selon au moins l'une des revendications précédentes, **caractérisée en ce que** la phase hydrophile de la préparation à deux phases contient en outre une ou plusieurs substances favorisant la conservation, de préférence du caprylylglycol.

9. Préparation selon au moins l'une des revendications précédentes, **caractérisée en ce que** la phase hydrophile de la préparation à deux phases contient du caprylylglycol en une teneur de 0,01 à 1,0% en poids, de préférence de 0,05 à 0,75% en poids, par rapport au poids total de la phase hydrophile.

10. Préparation selon au moins l'une des revendications précédentes, **caractérisée en ce que** le rapport pondéral du caprylylglycol au chlorure de benzéthonium est de 4,0:1 à 1:1.

11. Préparation selon au moins l'une des revendications précédentes, **caractérisée en ce que**, dans la phase lipidique de la préparation à deux phases, ladite au moins une huile végétale naturelle est choisie dans le groupe huile d'argan, huile d'avocat, huile de jojoba, huile d'olive, huile de tournesol, huile de soja, huile d'arachide, huile de colza, huile d'amande, huile de palmiste, huile de noix de coco, huile de ricin, huile de germes de blé, huile de pépins de raisin, huile de carthame, huile d'onagre et/ou huile de noix de macadamia, en particulier parmi l'huile d'avocat, l'huile de jojoba et/ou l'huile d'amande.

12. Préparation selon au moins l'une des revendications précédentes, **caractérisée en ce que** la phase lipidique de la préparation à deux phases contient ladite au moins une huile végétale en une teneur de 0,1 à 10,0% en poids, de préférence de 0,5 à 5,0% en poids, par rapport au poids total de la phase lipidique.

13. Préparation selon au moins l'une des revendications précédentes, **caractérisée en ce que** la phase lipidique de la préparation à deux phases contient en outre des huiles d'ester, de préférence des huiles d'ester choisies dans le groupe palmitate d'octyle, cocoate d'octyle, isostéarate d'octyle, dodécylmyristate d'octyle, isononanoate de cétéaryle, myristate d'isopropyle, palmitate d'isopropyle, stéarate d'isopropyle, oléate d'isopropyle, stéarate de n-butyle, laurate de n-hexyle, oléate de n-décyle, stéarate d'isooctyle, stéarate d'isononyle, isononanoate d'isononyle, palmitate de 2-éthylhexyle, laurate de 2-éthylhexyle, stéarate d'éthylhexyle, stéarate de 2-hexyldécyle, palmitate de 2-octyldodé-cyle, heptanoate de stéaryle, oléate d'oléyle, érucate d'oléyle, oléate d'érucyle, érucate d'érucyle et stéarate de tridécyle ainsi que leurs mélanges, de manière particulièrement préférée choisies parmi le stéarate d'éthylhexyle et/ou l'isononanoate de cétéaryle.

14. Préparation selon au moins l'une des revendications précédentes, **caractérisée en ce que** la quantité totale d'une ou de plusieurs huile(s) ester dans la phase lipidique de la préparation à deux phases est de 10,0 à 50,0% en poids, de préférence de 20 à 40% en poids, par rapport au poids total de la phase lipidique.

15. Préparation selon au moins l'une des revendications précédentes, **caractérisée en ce que** dans la phase lipidique de la préparation à deux phases contient en outre au moins un alcool monovalent saturé et/ou insaturé, ramifié et/ou non ramifié, présentant une longueur de chaîne de 3 à 30 atomes de carbone, de préférence au moins un alcool monovalent ramifié présentant une longueur de chaîne de 3 à 30 atomes de carbone, de manière particulièrement préférée de l'octyldodécanol.

16. Préparation selon au moins l'une des revendications précédentes, **caractérisée en ce que**, dans la phase lipidique de la préparation à deux phases, la teneur en au moins un alcool monovalent saturé et/ou insaturé, ramifié et/ou non ramifié présentant une longueur de chaîne de 3 à 30 atomes de carbone, est de 45,0 à 65,0% en poids, de préférence de 50,0 à 60,0% en poids, par rapport au poids total de la phase lipidique.

17. Préparation selon au moins l'une des revendications précédentes, **caractérisée en ce que**, dans la préparation à deux phases, la phase hydrophile et la phase lipidique se trouvent dans un rapport pondéral de phase hydrophile à phase lipidique de 1:3 à 3:1.

18. Utilisation de chlorure de benzéthonium conjointement avec du caprylylylglycol dans une phase hydrophile et d'une phase lipidique non conservée,
la phase hydrophile et la phase lipidique faisant partie d'une préparation à deux phases, pour la mise à disposition d'une préparation transparente à deux phases,
la préparation à deux phases présentant deux phases séparées qui peuvent être distinguées visuellement en une phase supérieure et une phase inférieure,
la phase hydrophile contenant
- au moins une substance de soin soluble dans l'eau,
- au moins un polyol choisi dans le groupe constitué par le glycérol et/ou le propylèneglycol et
- de l'eau et
la phase lipidique
- contenant au moins une huile végétale naturelle.
